# EUROPEAN PATENT SPECIFICATION

(11) **EP 2 702 036 B1**
(45) Date of publication and mention of the grant of the patent: **19.06.2019**
(21) Application number: 12718812.6
(22) Date of filing: 18.04.2012
(51) Int. Cl.: C07C 229/12, C11D 1/90

(54) **BETAINE ESTERS AND PROCESS FOR MAKING AND USING**
BETAINESTER SOWIE HERSTELLUNGS- UND VERWENDUNGSVERFAHREN
ESTERS DE BÉTAÏNE ET LEUR PROCÉDÉ DE FABRICATION ET D'UTILISATION

(30) Priority: 28.04.2011 US 201113096221
(43) Date of publication of application: 05.03.2014
(73) Proprietor: Eastman Chemical Company, Kingsport, TN 37660 (US)
(72) Inventor: BURK, Christopher, Harlan, Gray, TN 37615 (US); CLENDENNEN, Stephanie, Kay, Kingsport, TN 37663 (US); BOAZ, Neil, Warren, Kingsport, TN 37663 (US)
(74) Representative: Brown, Fraser Gregory James
(86) International application number: PCT/US2012/033983
(87) International publication number: WO 2012/148739

(56) References cited:
- ES-A1- 459 005
- JP-A- 6 067 122
- US-A- 3 360 550
- US-A1- 2006 116 290
- US-B1- 7 667 067
- KITANO H. ET AL.: "Image printing on the surface of anti-biofouling zwitterionic poplymer brishes by ion beam irradiation", MACROMOLECULAR BIOSCIENCE, vol. 11, 8 April 2011 (2011-04-08), pages 557-564, XP002678489,
- WENG W.-H. ET AL.: "A water-soluble amphoteric copolymer: synthesis and its disperssion properties on cement particles", JOURNAL OF APPLIED POLYMER SCIENCE, vol. 118, 2010, pages 1313-1319, XP002678490,
- CARR L R ET AL: "Functionalizable and nonfouling zwitterionic carboxybetaine hydrogels with a carboxybetaine dimethacrylate crosslinker", BIOMATERIALS, ELSEVIER SCIENCE PUBLISHERS BV., BARKING, GB, vol. 32, no. 4, 1 February 2011 (2011-02-01), pages 961-968, XP027514824, ISSN: 0142-9612, DOI: 10.1016/J.BIOMATERIALS.2010.09.067 [retrieved on 2010-10-20]
- DATABASE CA [Online] CHEMICAL ABSTRACTS SERVICE, COLUMBUS, OHIO, US; Wengui d. et al.: "Synthesis of new betaine-type amphoteric surfactants from dehydroabietic acid", XP002678491, Database accession no. 2005:1280259
- DATABASE CA [Online] CHEMICAL ABSTRACTS SERVICE, COLUMBUS, OHIO, US; Cui g.-Y. et al.: "Synthesis of betaine-type amphoteric surfactants based on natural rosin", XP002678492, Database accession no. 2007:1314346
- GANDHI N N: "APPLICATIONS OF LIPASE", JOURNAL OF THE AMERICAN OIL CHEMISTS' SOCIETY, SPRINGER, BERLIN, DE, vol. 74, no. 6, 1 June 1997 (1997-06-01), pages 621-634, XP000693786, ISSN: 0003-021X, DOI: 10.1007/S11746-997-0194-X
- DATABASE CA [Online] CHEMICAL ABSTRACTS SERVICE, COLUMBUS, OHIO, US; 2011, Wang L. et al.: "Synthesis of lauroylethyl betaine surfactant", XP002678383, Database accession no. 2011:1346379
- CAO Z. ET AL.: "Reversibly switching the function of a surface between attacking and defending against bacteria", ANGEWANDTE CHEMIE INTERNATIONAL EDITION ENGLISH, vol. 51, March 2012 (2012-03), pages 2602-2605, XP002678493,
- WALSH C. L. ET AL.: "Synthesis and characterization of novel zwitterionic lipids with pH-responsive biophysical properties", CHEMICAL COMMUNICATION, vol. 48, 11 April 2012 (2012-04-11), pages 5575-5577, XP002678494,
- Kanerva: "Studies on Chemo-and enantio-selectivity of the enzymatic monoacylations of aminoalcohols", Acta Chemica Scandinavica, 1 January 1992 (1992-01-01), pages 1101-1105, XP055313470, Retrieved from the Internet: URL:http://actachemscand.org/pdf/acta_vol_ 46_p1101-1105.pdf [retrieved on 2016-10-24]
- SCHERGNA S ET AL: "Enzymatic Modification of Fullerene Derivatives", TETRAHEDRON LETTERS, PERGAMON, GB, vol. 39, no. 42, 15 October 1998 (1998-10-15), pages 7791-7794, XP004134310, ISSN: 0040-4039, DOI: 10.1016/S0040-4039(98)01703-1
- COUTURIER L ET AL: "Lipase-catalyzed chemoselective aminolysis of various aminoalcohols with fatty acids", JOURNAL OF MOLECULAR CATALYSIS. B, ENZYMATIC, ELSEVIER, AMSTERDAM, NL, vol. 56, no. 1, 1 January 2009 (2009-01-01), pages 29-33, XP025675113, ISSN: 1381-1177, DOI: 10.1016/J.MOLCATB.2008.04.010 [retrieved on 2008-04-30]

## Description

### FIELD OF THE INVENTION

This invention pertains to betaine esters and processes for the preparation and use thereof.

### BACKGROUND OF THE INVENTION

There is an increasing industrial and societal need for the preparation of ingredients that reduce or eliminate organic solvents and irritants, employ reagents that are themselves biocompatible and that optimally use starting materials derived from a natural source or are "nature-equivalent." This is of urgent interest in consumer-facing industries such as personal and household care. One class of materials that might be approached in a "greener" manner is surfactants. In particular, there is a need for new betaines that are made in a more environmentally-friendly manner. Betaines are zwitterionic surfactants used in the personal care, household care, and other industries. They are classified as specialty co-surfactants that complement the performance of the primary surfactants. These co-surfactants also increase the mildness of the formulation by reducing irritation associated with purely ionic surfactants.

Betaines are commonly produced by a multi-step process based on coconut or palm kernel oil. For example, one process for the preparation of a prototypical betaine, fatty acid amidopropyl betaine, involves the amidation of fatty acids with 3-dimethylaminopropylamine (DMAPA) at high temperatures (150-175°C). The intermediate fatty aminoamide is then reacted with sodium chloroacetate to afford the final product. The amidation requires high temperatures for conversion and distillation to remove unreacted starting materials. These high reaction temperatures can generate by-products and impart color to the products, requiring additional steps to remove the by-products and the color. DMAPA is also a known sensitizer and is found in trace quantities in the final formulation. Thus, betaines prepared under mild conditions without the use of DMAPA would be of great interest.

ES 459 005 discloses compounds of general formula (R₃-Z-R₄)-N⁺(R₁)(R₂)-(CH₂)ₙCOOH and their salts, compositions therefof, preparation thereof and their use as surfactant.

Couturier L et al: "Lipase-Catalyzed Chemoselective Aminolysis Of Various Aminoalcohols With Fatty Acids", *Journal of Molecular Catalysis B: Enzymatic* 56.1 (2009): 29-33 describes the development of a solvent-free enzymatic process for the production of fatty alkanolamides.

US3360550 teaches a process of preparing a betaine derivative which is used as a germicide.

It would be highly desirable for the production of the betaines to occur under mild conditions and in high yield. Such a process would take place at lower temperatures, with fewer processing steps and by-products and it would lessen environmental impacts.

### BRIEF SUMMARY OF THE INVENTION

The present invention concerns a process for the preparation of betaine, comprising:
a) producing an ester of formula **2:** 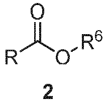
b) reacting a dialkylamino alcohol **3:** 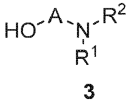 with **2** in the presence of an enzyme, Novozym 435 ™ (Candida Antartica lipase B immobilized on acrylic resin) to form an intermediate **4**: 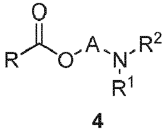
c) reacting intermediate **4** with sodium chloroacetate to produce a betaine.

wherein R is selected from the group consisting of branched and straight-chain, saturated, unsaturated and polyunsaturated C₁-C₂₂ hydrocarbyl, C₃-C₈ cycloalkyl, C₆-C₂₀ carbocyclic aryl, and C₄-C₂₀ heterocyclic wherein the heteroatoms are selected from the group consisting of sulfur, nitrogen, oxygen, and mixtures thereof,
R⁶ is a straight or branched chain C₁-C₆ alkyl,
R¹ and R² are the same or are independently selected from the group consisting of straight or banched-chain C₁-C₆ alkyl, C₂-C₆ alkenyl, C₄-C₆ dienyl, and C₃-C₈ cycloalkyl, and
A is selected from the group consisting of branched and straight-chain, saturated, unsaturated and polyunsaturated C₁-C₁₀ divalent hydrocarbyl, C₃-C₈ cycloalkylene, C₆-C₁₀ carbocyclic arylene, and C₄-C₁₀ divalent heterocyclic wherein the heteroatoms are selected from sulfur, nitrogen, and oxygen;
and wherein said hydrocarbyl group in R and/or said divalent hydrocarbyl group in A may be substituted with one to five groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen;
and wherein said alkyl, alkenyl and dienyl groups in R¹ and R² may be substituted with one to three groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen, wherein the branching and/or substitution of R¹ and R² may connect to form a ring.

### DETAILED DESCRIPTION

An embodiment concerns a process for the preparation of betaines. The first step of the process is the production of esters of the general formula **2:** 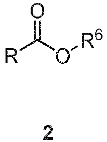 wherein R is selected from substituted and unsubstituted, branched- and straight-chain, saturated, unsaturated, and polyunsaturated C₁-C₂₂ hydrocarbyl, substituted and unsubstituted C₃-C₈ cycloalkyl, substituted and unsubstituted C₆-C₂₀ carbocyclic aryl, and substituted and unsubstituted C₄-C₂₀ heterocyclic wherein the heteroatoms are selected from sulfur, nitrogen, and oxygen, or mixtures thereof, and R⁶ may be C₁-C₆ straight or branched chain alkyl.

The saturated, unsaturated, and polyunsaturated alkyl groups which may be represented by R may be straight- or branched-chain hydrocarbon radicals containing up to about 22 carbon atoms and may be substituted, for example, with one to five groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen. The terms "C₁-C₆-alkoxy", "C₂-C₇-alkoxycarbonyl", and "C₂-C₇-alkanoyloxy" are used to denote radicals corresponding to the structures -OR³, -CO₂R³, and -OCOR³, respectively, wherein R³ is C₁-C₆-alkyl or substituted C₁-C₆-alkyl. The terms "C₂-C₁₆ aminocarbonyl" and "C₂-C₁₆ amido" are used to denote radicals corresponding to the structures -NHCOR⁴, -CONHR⁴, respectively, wherein R⁴ is C₁-C₁₅-alkyl or substituted C₁-C₁₅-alkyl. The term "C₃-C₈-CyCloalkyl" is used to denote a saturated, carbocyclic hydrocarbon radical having three to eight carbon atoms.

The aryl groups which R may represent (or any aryl substituents) may include phenyl, naphthyl, or anthracenyl and phenyl, naphthyl, or anthracenyl substituted with one to five substituents selected from C₁-C₆-alkyl, substituted C₁-C₆-alkyl, C₆-C₁₀ aryl, substituted C₆-C₁₀ aryl, C₁-C₆-alkoxy, halogen, carboxy, cyano, C₂-C₇-alkanoyloxy, C₁-C₆-alkylthio, C₁-C₆-alkylsulfonyl, trifluoromethyl, hydroxy, C₂-C₇-alkoxycarbonyl, C₂-C₇-alkanoylamino and - OR⁵, -S-R⁵, -SO₂-R⁵, -NHSO₂R⁵ and -NHCO₂R⁵, wherein R⁵ is phenyl, naphthyl, or phenyl or naphthyl substituted with one to three groups selected from C₁-C₆-alkyl, C₆-C₁₀ aryl, C₁-C₆-alkoxy and halogen.

The heterocyclic groups which R may represent (or any heteroaryl substituents) include 5- or 6- membered ring containing one to three heteroatoms selected from oxygen, sulfur and nitrogen. Examples of such heterocyclic groups are pyranyl, oxopyranyl, dihydropyranyl, oxodihydropyranyl, tetrahydropyranyl, thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, pyridyl, pyrimidyl, benzoxazolyl, benzothiazolyl, benzimidazolyl, indolyl and the like. The heterocyclic radicals may be substituted, for example, with up to three groups such as C₁-C₆-alkyl, C₁-C₆-alkoxy, substituted C₁-C₆-alkyl, halogen, C₁-C₆-alkylthio, aryl, arylthio, aryloxy, C₂-C₇-alkoxycarbonyl and C₂-C₇-alkanoylamino. The heterocyclic radicals also may be substituted with a fused ring system, e.g., a benzo or naphtho residue, which may be unsubstituted or substituted, for example, with up to three of the groups set forth in the preceding sentence.

Short chain esters **2** can be produced by any practical method, including the solvolysis of triglycerides in the presence of a C₁-C₄ alcohol and a base, acid or enzyme catalyst as is known in the art. Examples of C₁-C₄ alcohols include methanol, ethanol, 1-propanol, 2-propanol, 1-butanol, 2-butanol, and isobutanol. The short-chain esters **2** may contain from 0-20% of residual lower alcohol.

The second step comprises the enzymatic reaction of a dialkylamino alcohol **3:** 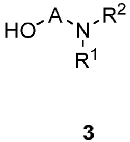 with **2** in the presence of an enzyme Novozym 435™_(Candida Antartica lipase B immobilized on acrylic resin) with or without methods for the removal of the alcohol by-product to form the desired intermediate **4**, 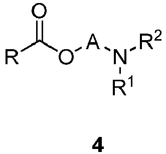 wherein R is defined above, and R¹ and R² are selected from straight or branched chain C₁-C₆ alkyl, C₂-C₆ alkenyl or C₄-C₆ dienyl, and A is selected from branched and straight chain C₁-C₈ alkylene, branched- and straight-chain saturated C₂-C₈ alkenylene, substituted and unsubstituted C₃-C₈ cycloalkylene, substituted and unsubstituted C₆-C₁₀ carbocyclic arylene, substituted and unsubstituted C₄-C₁₂ divalent heterocyclic, or mixtures thereof.

The alkyl, alkenyl and dienyl groups which may be represented by R¹ and R² may be straight- or branched-chain hydrocarbon radicals containing up to about 6 carbon atoms and may be substituted, for example, with one to three groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen. The terms "C₁-C₆-alkoxy", "C₂-C₇-alkoxycarbonyl", and "C₂-C₇-alkanoyloxy" are used to denote radicals corresponding to the structures -OR³, -CO₂R³, and -OCOR³, respectively, wherein R³ is C₁-C₆-alkyl or substituted C₁-C₆-alkyl. The terms "C₂-C₁₆ aminocarbonyl" and "C₂-C₁₆ amido" are used to denote radicals corresponding to the structures -NHCOR⁴, -CONHR⁴, respectively, wherein R⁴ is C₁-C₁₅-alkyl or substituted C₁-C₁₅-alkyl. The term "C₃-C₈-cycloalkyl" is used to denote a saturated, carbocyclic hydrocarbon radical having three to eight carbon atoms.

The divalent hydrocarbyl radicals which may be represented by A may be straight- or branched-chain saturated, unsaturated, and polyunsaturated alkylene and cycloalkylene groups containing up to about 10 carbon atoms and may be substituted, for example, with one to five groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen. The terms "C₁-C₆-alkoxy", "C₂-C₇-alkoxycarbonyl", and "C₂-C₇-alkanoyloxy" are used to denote radicals corresponding to the structures -OR³, -CO₂R³, and - OCOR³, respectively, wherein R³ is C₁-C₆-alkyl or substituted C₁-C₆-alkyl. The terms "C₂-C₁₆ aminocarbonyl" and "C₂-C₁₆ amido" are used to denote radicals corresponding to the structures -NHCOR⁴, -CONHR⁴, respectively, wherein R⁴ is C₁-C₁₅-alkyl or substituted C₁-C₁₅-alkyl.

The arylene groups which A may represent may include phenylene, naphthylene, or anthracenylene and phenylene, naphthylene, or anthracenylene substituted with one to five substituents selected from C₁-C₆-alkyl, substituted C₁-C₆-alkyl, C₆-C₁₀ aryl, substituted C₆-C₁₀ aryl, C₁-C₆-alkoxy, halogen, carboxy, cyano, C₂-C₇-alkanoyloxy, C₁-C₆-alkylthio, C₁-C₆-alkylsulfonyl, trifluoromethyl, hydroxy, C₂-C₇-alkoxycarbonyl, C₂-C₇-alkanoylamino and -OR⁵, -S-R⁵, -SO₂-R⁵, -NHSO₂R⁵ and -NHCO₂R⁵, wherein R⁵ is phenyl, naphthyl, or phenyl or naphthyl substituted with one to three groups selected from C₁-C₆-alkyl, C₆-C₁₀ aryl, C₁-C₆-alkoxy and halogen.

The divalent heterocyclic groups which A may represent include 5- or 6- membered ring containing one to three heteroatoms selected from oxygen, sulfur and nitrogen. Examples of such heterocyclic groups are pyranyl, oxopyranyl, dihydropyranyl, oxodihydropyranyl, tetrahydropyranyl, thienyl, furyl, pyrrolyl, imidazolyl, pyrazolyl, thiazolyl, isothiazolyl, oxazolyl, isoxazolyl, triazolyl, thiadiazolyl, oxadiazolyl, tetrazolyl, pyridyl, pyrimidyl, benzoxazolyl, benzothiazolyl, benzimidazolyl, indolyl and the like. The heterocyclic radicals may be substituted, for example, with up to three groups such as C₁-C₆-alkyl, C₁-C₆-alkoxy, substituted C₁-C₆-alkyl, halogen, C₁-C₆-alkylthio, aryl, arylthio, aryloxy, C₂-C₇-alkoxycarbonyl and C₂-C₇-alkanoylamino. The heterocyclic radicals also may be substituted with a fused ring system, e.g., a benzo or naphtho residue, which may be unsubstituted or substituted, for example, with up to three of the groups set forth in the preceding sentence.

The term "halogen" is used to include fluorine, chlorine, bromine, and iodine.

The process of step two is carried out without solvent or in an inert solvent chosen from cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile, dimethyl formamide, or dimethyl sulfoxide, or mixtures thereof.

The process may be carried out at a temperature from about -100 °C to about the boiling point of the solvent, from about 20 to about 80 °C, or from about 50 to about 70 °C. The amount of alcohol **3** may be from about 0.85 to about 20 equivalents based on the ester **2**, or can be from about 1 to about 10 equivalents, or even from about 1 to about 1.5 equivalents. The use of short chain alcohol esters of carboxylic acids is beneficial to the success of the enzymatic esterification of the amino alcohol. Unmodified carboxylic acids may be used in the enzymatic esterification, however the acid forms a salt with the amino alcohol and limits the efficiency of the reaction.

The enzyme used in the process is Novozym 435™ (Candida antarctica lipase B immobilized on acrylic resin).

Removal of the alcohol or water byproducts can be done chemically via an alcohol or water absorbent (e.g., molecular sieves) or by physical removal of the alcohol or water. According to an embodiment, this by-product removal can be done by evaporation, either by purging the reaction mixture with an inert gas such as nitrogen, argon, or helium, or by performing the reaction at reduced pressures, or both, as these conditions can afford >98% conversion of ester **2** to intermediate **4.** According to an embodiment, pressure for the reaction is from about 1 torr to about ambient pressure, or from about 50 torr to about ambient pressure. Any organic solvent that is included in this process may or may not be removed along with the alcohol or water. Examples of **3** include dimethylaminoethanol and dimethylaminopropanol.

The third step to generate the final product **1** comprises the reaction of intermediate **4** with sodium chloroacetate. 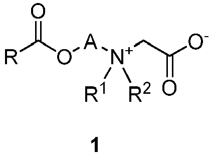 The process is carried out without solvent or in an inert solvent chosen from water, cyclic or acyclic alcohol solvents such as methanol, ethanol, 1-propanol, 2-propanol, 1-butanol, 2-butanol, isobutanol, ethylene glycol, 1,2-propanediol, or 1,3-propanediol, cyclic or acyclic ether solvents such as diethyl ether, diisopropyl ether, tert-butyl methyl ether, or tetrahydrofuran, aromatic hydrocarbons such as benzene, toluene, or xylene, aliphatic or alicyclic saturated or unsaturated hydrocarbons such as hexane, heptane, cyclohexane, or limonene, halogenated hydrocarbons such as dichloromethane, dichloroethane, dibromoethane, tetrachloroethylene, or chlorobenzene, polar aprotic solvents such as acetonitrile, dimethyl formamide, or dimethyl sulfoxide, or mixtures thereof. The preferred solvents are water, alcohols, no solvent or mixtures thereof. The process may be carried out at a temperature of from about -100 °C to about the boiling point of the solvent, from about 25 to about 150 °C, or from about 50 to about 100 °C. The amount of sodium chloroacetate may be from about 0.75 to about 20 equivalents based on **4**, from about 1 to about 10 equivalents, or from about 1 to about 1.5 equivalents. If included, a base is chosen from metal hydroxides or metal carbonates. According to an embodiment, bases can be sodium hydroxide and potassium hydroxide. The amount of base can be from about 0 molar equivalents to about 1 molar equivalent based on ester **4** or in an amount high enough to keep the reaction mixture basic, for example at about pH 8-9.

The intermediate **4** and the product **1** of the process may be isolated using methods known to those of skill in the art, e.g., extraction, filtration, or crystallization.

Also disclosed is the use of the betaine esters **1** as surfactants. The surfactant properties of the betaine esters **1** can be determined by a number of tests including an ASTM foam height test and a test for critical micelle concentration.

The Standard Test Method for Foaming Properties of Surface-Active Agents (ASTM 1173-07) was used to determine the foaming properties of the betaine esters **1** described herein. This method generates foam under low-agitation conditions and is generally used for moderate- and high-foam surfactants. This test gathers data on initial foam height and foam decay. Foam decay provides information on foam stability.

The apparatus for carrying out this test includes a jacketed column and a pipet. The jacketed column serves as a receiver, while the pipet delivers the surface-active solution. Solutions of each surface-active agent were prepared. The betaine solution to be tested was added to the receiver (50 mL) and to the pipet (200 mL). The pipet was positioned above the receiver and opened. As the solution fell and made contact with the solution in the receiver, foam was generated. When the pipet was empty, the time was noted and an initial foam height was recorded. The foam height was recorded each minute for five minutes. Exact size specifications for the glassware can be found in ASTM 1173-07.

**Table 1**

| **Example No.** | **Foam height (cm) at time t (min)** | | | | | | | | | | | |
|---|---|---|---|---|---|---|---|---|---|---|---|---|
| | 1 g/L (0.1 weight %) | | | | | | 10 g/L (1.0 weight %) | | | | | |
| | t=0 | 1 | 2 | 3 | 4 | 5 | t=0 | 1 | 2 | 3 | 4 | 5 |
| **4** | 9.0 | 9.0 | 9.0 | 9.0 | 9.0 | 9.0 | 16.5 | 16.5 | 16.0 | 16.0 | 16.0 | 16.0 |
| **5** | 15.0 | 14.0 | 14.0 | 13.5 | 13.5 | 13.5 | 17.0 | 16.5 | 16.0 | 15.5 | 15.5 | 15.0 |
| **6** | 16.0 | 15.5 | 15.5 | 15.5 | 15.5 | 15.5 | 15.0 | 15.0 | 15.0 | 15.0 | 15.0 | 15.0 |
| **8** | 14.0 | 13.5 | 13.5 | 13.5 | 13.0 | 13.0 | 17.0 | 16.0 | 15.5 | 15.5 | 15.0 | 15.0 |
| **9** | 15.5 | 15.0 | 15.0 | 14.5 | 14.5 | 14.0 | 17.0 | 16.0 | 15.5 | 15.5 | 15.5 | 15.0 |
| **11** | 10.0 | 10.0 | 10.0 | 10.0 | 9.5 | 9.5 | 21.0 | 19.5 | 19.0 | 19.0 | 18.5 | 18.5 |
| **Comparative example no.** | | | | | | | | | | | | |
| **2** | 17.0 | 16.5 | 16.5 | 16.0 | 16.0 | 16.0 | 17.5 | 17.0 | 17.0 | 16.5 | 16.5 | 16.5 |
| **4** | 15.5 | 15.5 | 15.5 | 15.5 | 15.5 | 15.5 | 16.5 | 16.0 | 15.5 | 15.5 | 15.5 | 15.5 |
| **6** | 16.5 | 16.0 | 15.5 | 15.5 | 15.5 | 15.5 | 17.5 | 17.0 | 16.5 | 16.5 | 16.0 | 15.5 |
| **8** | 16.0 | 15.0 | 15.0 | 14.0 | 12.0 | 5.0 | 17.0 | 15.5 | 14.0 | 13.0 | 7.0 | 5.0 |

Data from the foam height test can be found in **Table 1.** Examples 4-6, 8, 9, and 11 are betaine esters, while Comparative Examples 2, 4, 6 and 8 are betaine amides for comparison. These compounds were prepared at 1 g/L and 10 g/L solutions. As the data in **Table 1** indicate, solutions of the betaine esters generate large amounts of foam. Examples in which foam height does not decrease over time indicate good foam stability. Comparative Example 2 is a useful standard, in that this compound is used commercially as a betaine surfactant.

The critical micelle concentration (CMC) was also determined for each compound. The CMC is the concentration of surfactants above which micelles spontaneously form. CMC is an important characteristic of a surfactant. At surfactant concentrations below the CMC, surface tension varies widely with surfactant concentration. At concentrations above the CMC, surface tension remains fairly constant. A lower CMC indicates less surfactant is needed to saturate interfaces and form micelles. Typical CMC values for surface-active agents are less than 1 weight %.

The fluorimetric determination of CMC described by Chattopadhyay and London (Analytical Biochemistry, 139, 408-412, 1984) was used to obtain the critical micelle concentrations found in **Table 2.** This method employs the fluorescent dye 1,6-diphenyl-1,3,5-hexatriene (DPH) in a solution of the surface-active agent. The analysis is based on differences in fluorescence upon incorporation of the dye into the interior of the micelles. As the solution exceeds CMC, a large increase in fluorescence intensity is observed. This method has been found to be sensitive and reliable, and has been demonstrated on zwitterionic, anionic, cationic and uncharged surface-active agents.

**Table 2**

| **Example No.** | **CMC** (weight %) |
|---|---|
| 4 | 0.0050 |
| 5 | 0.0053 |
| 6 | 0.0007 |
| 8 | 0.0045 |
| 9 | 0.0023 |
| 11 | 0.0004 |

| **Comparative Example No.** | |
|---|---|
| 2 | 0.0029 |
| 4 | 0.0041 |
| 6 | 0.0025 |
| 8 | 0.0027 |

The data in **Table 2** indicate that very low concentrations of the betaine esters are needed to reach CMC. Again, Examples 4-6, 8, 9, and 11 are betaine esters, while Comparative Examples 2, 4, 6 and 8 are betaine amides for comparison. As with foam height, all of these compounds appear similar. These values fall in the range of being useful as surface-active agents. As noted above, Comparative Example 2 is used commercially as a betaine surfactant and provides a reference point by which to compare values for the betaine esters **1.**

The betaine esters are molecules possessing both hydrophilic and hydrophobic regions, making them useful as surfactants in a number of formulated product applications, including personal care products such as skin care, hair care or other cosmetic products, household and industrial surface cleaners, disinfectants, metal working, rust inhibitors, lubricants, agrochemicals, and dye dispersions. Betaines can also be used as emulsifiers and thickening agents in emulsions. Betaines are often formulated into products as secondary surface-active agents. Although a primary use is as humectants and foaming agents, betaines are also used for their anti-static and viscosity-controlling properties.

Such product formulations can contain from about 0.001 weight % to about 20 weight %, from about 0.01 weight % to about 15 weight %, or even from about 0.1 weight % to about 10 weight % of the betaine esters.

Product formulations disclosed may include other surfactants in addition to the betaine esters. These surfactants can include anionic surfactants (such as alcohol ether sulfates, linear alkylbenzene sulfonates, acyl isethionates), cationic surfactants (such as quaternary ammonium salts, fatty amine oxides, and ester quats), and non-ionic surfactants (such as alky polyglycosides, alcohol ethoxylates, and fatty alcanol amides). Such ingredients are known to those of skill in the art.

The cosmetic, skin, and hair care compositions disclosed may also contain other skin conditioning ingredients or cosmetically acceptable carriers in addition to the betaine esters.

Such formulations may also contain skin care ingredients/carriers such as retinol, retinyl esters, tetronic acid, tetronic acid derivatives, hydroquinone, kojic acid, gallic acid, arbutin, α-hydroxy acids, niacinamide, pyridoxine, ascorbic acid, vitamin E and derivatives, aloe, salicylic acid, benzoyl peroxide, witch hazel, caffeine, zinc pyrithione, and fatty acid esters of ascorbic acid. Such other ingredients are known to those of skill in the art.

Other ingredients that may be included in these formulations include conditioning agents (such as polyquaterniums and panthenol), pearlizing agents (such as glycol distearate, distearyl ether, and mica), UV filters (such as octocrylene, octyl methoxycinnamate, benzophenone-4, titanium dioxide, and zinc oxide), exfoliation additives (such as apricot seeds, walnut shells, polymer beads, and pumice), silicones (such as dimethicone cyclomethicone, and amodimethicone), moisturizing agents (such as petrolatum, sunflower oil, fatty alcohols, and shea butter), foam stabilizers (such as cocamide MEA and cocamide DEA), anti-bacterial agents such as triclosan, humectants such as glycerin, thickening agents (such as guar, sodium chloride, and carbomer), hair and skin damage repair agents (such as proteins, hydrolyzed proteins, and hydrolyzed collagen), and foam boosters such as cocamide MIPA. Such other ingredients are known to those of skill in the art.

Many preparations are known in the art, and include formulations containing acceptable carriers such as water, oils and/or alcohols and emollients such as olive oil, hydrocarbon oils and waxes, silicone oils, other vegetable, animal or marine fats or oils, glyceride derivatives, fatty acids or fatty acid esters or alcohols or alcohol ethers, lecithin, lanolin and derivatives, polyhydric alcohols or esters, wax esters, sterols, phospholipids and the like. These same general ingredients can be formulated into liquids (such as liquid soaps, shampoos, or body washes), creams, lotions, gels, or into solid sticks by utilization of different proportions of the ingredients and/or by inclusion of thickening agents such as gums or other forms of hydrophilic colloids.

### Examples

The processes provided by the present invention are further illustrated by the following examples.

### Example 1

### Preparation of Methyl Cocoate

To a jar was added potassium hydroxide (1 g) and methanol (25 g). The solution was stirred for 1 hour. To a separate jar was added coconut oil (100 g). The solid was heated to a melt and the KOH/MeOH solution was added and the mixture was stirred overnight. The mixture was transferred to a separatory funnel and allowed to separate. The bottom (glycerol) layer was removed. The top layer was filtered to afford a pale yellow oil (100 g). ¹H NMR (300 MHz, CDCl₃) δ 3.65 (s, 3H), 2.28 (t, 2H), 1.60 (m, 2H), 1.24 (s, 16H), 0.86 (t, 3H).

### Example 2

### Preparation of Ethyl Cocoate

To a jar was added potassium hydroxide (2 g) and ethanol (72 g). The solution was stirred for 1 hour. To a separate jar was added coconut oil (200 g). The solid was heated to a melt and the KOH/EtOH solution was added and the mixture was stirred overnight. The mixture was transferred to a separatory funnel and allowed to separate. The bottom (glycerol) layer was removed. The top layer was filtered to afford a pale yellow oil (227 g). ¹H NMR (300 MHz, CDCl₃) δ 4.09 (t, 3H), 3.68 (q, 2H), 2.27 (t, 2H), 1.60 (m, 2H), 1.24 (s, 16H), 0.86 (t, 3H).

### Example 3

### Preparation of Dimethylaminoethyl Cocoate

To a 50 mL conical bottom plastic vial was added ethyl cocoate (10 g, 38.5 mmol), dimethylaminoethanol (5.09 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (8 g) without further purification. ¹H NMR (300 MHz, CDCl₃) δ 4.15 (t, 2H), 2.54 (t, 2H), 2.31 (t, 2H), 2.26 (s, 6H), 1.60 (m, 2H), 1.24 (s, 16H), 0.86 (t, 3H).

### Example 4

### Preparation of Dimethylaminoethyl Cocoate Betaine

To a 100 mL round bottom flask with a magnetic stir bar and a condenser was added dimethylaminoethyl cocoate (10 g, 35.3 mmol), sodium chloroacetate (4.11 g, 35.3 mmol, 1eq) and water (32.9 g). The reaction mixture was heated at 98 °C for 8 hours. The pH was kept basic by the addition of 50% NaOH. When the reaction was complete, the mixture was neutralized with 1 M HCI and allowed to cool. The reaction mixture was filtered to afford the product as a 30% aqueous solution (43 g). ¹H NMR (300 MHz, DMSO d-6) δ 3.89 (t, 2H), 3.78 (t, 2H), 3.66 (s, 2H), 3.17 (s, 6H), 2.27 (t, 2H), 1.51 (m, 2H), 1.23 (s, 16H), 0.85 (t, 3H).

### Example 5

### Preparation of Dimethylaminoethyl Cocoate Betaine

To a 100 mL round bottom flask with a magnetic stir bar and a condenser was added dimethylaminoethyl cocoate (10 g, 35.3 mmol), sodium chloroacetate (4.11 g, 35.3 mmol, 1eq) and 1,3-propanediol (4.7 g). The reaction mixture was heated at 98 °C for 8 hours. When the reaction was complete by NMR, the mixture was allowed to cool. The mixture was filtered to afford the product as a viscous, 75% solution in 1,3-propanediol (14 g). ¹H NMR (300 MHz, DMSO *d*-6) δ 3.89 (t, 2H), 3.78 (t, 2H), 3.66 (s, 2H), 3.17 (s, 6H), 2.27 (t, 2H), 1.51 (m, 2H), 1.23 (s, 16H), 0.85 (t, 3H).

### Example 6

### Preparation of Dimethylaminoethyl Cocoate Betaine

To a 100 mL round bottom flask with a magnetic stir bar and a condenser was added dimethylaminoethyl cocoate (10 g, 35.3 mmol), sodium chloroacetate (4.11 g, 35.3 mmol, 1eq) and isopropanol (15 mL). The reaction mixture was heated at reflux for 8 hours. When the reaction was complete by NMR, the mixture was allowed to cool. The mixture was filtered and isopropanol was removed *in vacuo* to afford the product as a viscous, semi-solid (13 g). ¹H NMR (300 MHz, DMSO *d*-6) δ 3.89 (t, 2H), 3.78 (t, 2H), 3.66 (s, 2H), 3.17 (s, 6H), 2.27 (t, 2H), 1.51 (m, 2H), 1.23 (s, 16H), 0.85 (t, 3H).

### Example 7

### Preparation of Dimethylaminopropyl Cocoate

To a 50 mL conical bottom plastic vial was added ethyl cocoate (10 g, 38.5 mmol), dimethylaminopropanol (4.76 g, 46.2 mmol, 1.2 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (9.2 g) without further purification. ¹H NMR (300 MHz, CDCl₃) δ 4.10 (t, 2H), 2.30 (m, 4H), 2.21 (s, 6H), 1.78 (t, 2H), 1.60 (m, 2H), 1.24 (s, 16H), 0.86 (t, 3H).

### Example 8

### Preparation of Dimethylaminopropyl Cocoate Betaine

To a 100 mL round bottom flask with a magnetic stir bar and a condenser was added dimethylaminopropyl cocoate (10 g, 35 mmol), sodium chloroacetate (4.1 g, 35 mmol, 1eq) and 1,3-propanediol (14.1 g). The reaction mixture was heated at 98 °C for 8 hours. When the reaction was complete by NMR, the mixture was allowed to cool. The mixture was filtered to afford the product as a 50% solution in 1,3-propanediol (27 g). ¹H NMR (300 MHz, CDCl₃) δ 4.16 (t, 2H), 3.92 (t, 2H), 3.67 (t, 2H), 3.28 (s, 6H), 2.34 (q, 2H), 2.10 (t, 2H), 1.60 (m, 2H), 1.26 (s, 16H), 0.88 (t, 3H).

### Example 9

### Preparation of Dimethylaminopropyl Cocoate Betaine

To a 100 mL round bottom flask with a magnetic stir bar and a condenser was added dimethylaminopropyl cocoate (10 g, 35.3 mmol, 1eq), sodium chloroacetate (4.11 g, 35.3 mmol, 1eq) and isopropanol (15 mL). The reaction mixture was heated at reflux for 8 hours. When the reaction was complete by NMR, the mixture was allowed to cool. The mixture was filtered and isopropanol was removed *in vacuo* to afford the product as a viscous, semi-solid (14 g). ¹H NMR (300 MHz, CDCl₃) δ 4.16 (t, 2H), 3.92 (t, 2H), 3.67 (t, 2H), 3.28 (s, 6H), 2.34 (q, 2H), 2.10 (t, 2H), 1.60 (m, 2H), 1.26 (s, 16H), 0.88 (t, 3H).

### Example 10

### Preparation of Dimethylamino-2-methylethyl Cocoate

To a 50 mL conical bottom plastic vial was added ethyl cocoate (10 g, 38.5 mmol), dimethylamino-2-methylpropanol (5.95 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (7 g) without further purification. ¹H NMR (300 MHz, CDCl₃) δ 5.01 (m, 1H), 2.61 (t, 2H), 2.31 (t, 2H), 2.29 (m, 7H), 1.60 (m, 2H), 1.24 (m, 19H), 0.86 (t, 3H).

### Example 11

### Preparation of Dimethylamino-2-methylethyl Cocoate Betaine

To a 100 mL round bottom flask with a magnetic stir bar and a condenser was added dimethylamino-2-methylethyl cocoate (5.6 g, 18.8 mmol), sodium chloroacetate (2.18 g, 18.8 mmol, 1eq) and water (7.8 g). The reaction mixture was heated at 98 °C for 8 hours. The pH was kept basic by the addition of 50% NaOH. When the reaction was complete, the mixture was neutralized with 1 M HCl and allowed to cool. The reaction mixture was filtered to afford the product as a 50% solution in water (14 g). ¹H NMR (300 MHz, DMSO *d*-6) δ 4.96 (m, 1H), 3.89 (t, 2H), 3.66 (s, 2H), 3.17 (s, 6H), 2.27 (t, 2H), 1.51 (m, 2H), 1.23 (m, 19H), 0.85 (t, 3H).

### Comparative Example 1

### Preparation of Dimethylaminopropyl Cocoamide

To a 50 mL conical bottom plastic vial was added ethyl cocoate (10 g, 38.5 mmol), dimethylaminopropylamine (5.9 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (8.9 g) without further purification. ¹H NMR (300 MHz, CDCl₃) δ 7.02 (s, 1H), 3.28 (m, 2H), 2.32 (m, 2H), 2.18 (s, 6H), 2.10 (t, 2H), 1.59 (m, 4H), 1.21 (s, 16H), 0.84 (t, 3H).

### Comparative Example 2

### Preparation of Dimethylaminopropyl Cocoamide Betaine

To a 100 mL round bottom flask with a magnetic stir bar and a condenser was added dimethylaminopropyl cocoamide (10 g, 35 mmol), sodium chloroacetate (4.1 g, 35 mmol, 1eq) and water (14.7 g). The reaction mixture was heated at 98 °C for 8 hours. The pH was kept basic by the addition of 50% NaOH. When the reaction was complete, the mixture was neutralized with 1 M HCl and allowed to cool. The reaction mixture was filtered to afford the product as a 45% solution in water (33 g). ¹H NMR (300 MHz, DMSO *d*-6) δ 8.07 (s, 1H), 3.59 (s, 2H), 3.45 (m, 2H), 3.08 (s, 6H), 3.05 (m, 2H), 2.04 (t, 2H), 1.76 (m, 2H), 1.44 (m, 2H), 1.19 (s, 16H), 0.81 (t, 3H).

### Comparative Example 3

### Preparation of Diethylaminopropyl Cocoamide

To a 50 mL conical bottom plastic vial was added ethyl cocoate (10 g, 38.5 mmol), diethylaminopropylamine (7.52 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (11 g) without further purification. ¹H NMR (300 MHz, CDCl₃) δ 7.45 (s, 1H), 3.29 (m, 2H), 2.47 (m, 6H), 2.08 (m, 2H), 1.58 (m, 4H), 1.23 (s, 16H), 0.99 (m, 6H), 0.84 (t, 3H).

### Comparative Example 4

### Preparation of Diethylaminopropyl Cocoamide Betaine

To a 100 mL round bottom flask with a magnetic stir bar and a condenser was added diethylaminopropyl cocoamide (5 g, 16 mmol), sodium chloroacetate (1.85 g, 16 mmol, 1eq) and water (5.85 g). The reaction mixture was heated at 98 °C for 8 hours. The pH was kept basic by the addition of 50% NaOH. When the reaction was complete, the mixture was neutralized with 1 M HCI and allowed to cool. The reaction mixture was filtered to afford the product as a 38% solution in water (11 g). ¹H NMR (300 MHz, DMSO d-6) δ 8.05 (s, 1H), 3.58 (s, 2H), 3.06 (q, 2H), 2.86 (m, 6H), 2.04 (t, 2H), 1.68 (m, 2H), 1.44 (m, 2H), 1.20 (s, 16H), 1.10 (t, 6H), 0.82 (t, 3H).

### Comparative Example 5

### Preparation of Dimethylaminoethyl Cocoamide

To a 50 mL conical bottom plastic vial was added ethyl cocoate (10 g, 38.5 mmol), dimethylaminoethylamine (5.09 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as a pale yellow oil (8.6 g) without further purification. ¹H NMR (300 MHz, CDCl₃) δ 6.25 (s, 1H), 3.25 (m, 2H), 2.34 (t, 2H), 2.16 (s, 6H), 2.10 (t, 2H), 1.54 (m, 2H), 1.18 (s, 16H), 0.80 (t, 3H).

### Comparative Example 6

### Preparation of Dimethylaminoethyl Cocoamide Betaine

To a 100 mL round bottom flask with a magnetic stir bar and a condenser was added dimethylaminoethyl cocoamide (8 g, 28.3 mmol), sodium chloroacetate (3.3 g, 28.3 mmol, 1eq) and water (11 g). The reaction mixture was heated at 98 °C for 8 hours. The pH was kept basic by the addition of 50% NaOH. When the reaction was complete, the mixture was neutralized with 1 M HCl and allowed to cool. The reaction mixture was filtered to afford the product as a 50% solution in water (21 g). ¹H NMR (300 MHz, DMSO *d*-6) δ 8.33 (t, 1H), 3.65 (s, 2H), 3.61 (m, 2H), 3.42 (q, 2H), 3.14 (s, 6H), 2.06 (t, 2H), 1.45 (m, 2H), 1.20 (s, 16H), 0.83 (t, 3H).

### Comparative Example 7

### Preparation of Diethylaminoethyl Cocoamide

To a 50 mL conical bottom plastic vial was added ethyl cocoate (10 g, 38.5 mmol), diethylaminoethylamine (6.71 g, 57.7 mmol, 1.5 eq) and Novozym 435™ (400 mg). A syringe was inserted through the cap and two additional holes were punched for gas to exit. Nitrogen was bubbled at a rate sufficient to mix the contents. The vial was placed in a heating block set to 65 °C. The reaction was monitored by GC/MS to observe the disappearance of starting material. The reaction was complete after approximately 24 hours. The reaction mixture was allowed to cool. The Novozym 435™ was removed by filtration to afford the product as_a pale yellow oil (10.2 g) without further purification. ¹H NMR (300 MHz, CDCl₃) δ 6.21 (s, 1H), 3.32 (m, 2H), 2.56 (m, 6H), 2.21 (m, 2H), 1.65 (m, 2H), 1.29 (s, 16H), 1.04 (m, 6H), 0.92 (t, 3H).

### Comparative Example 8

### Preparation of Diethylaminoethyl Cocoamide Betaine

To a 100 mL round bottom flask with a magnetic stir bar and a condenser was added diethylaminoethyl cocoamide (5 g, 16.7 mmol), sodium chloroacetate (1.94 g, 16.7 mmol, 1 eq) and water (14.7 g). The reaction mixture was heated at 98 °C for 8 hours. The pH was kept basic by the addition of 50% NaOH. When the reaction was complete, the mixture was neutralized with 1 M HCl and allowed to cool. The reaction mixture was filtered to afford the product as a 38% solution in water (18 g). ¹H NMR (300 MHz, DMSO *d*-6) δ 8.01 (s, 1H), 3.54 (s, 2H), 3.20 (q, 2H), 2.70 (m, 6H), 2.04 (t, 2H), 1.45 (t, 2H), 1.21 (s, 16H), 1.03 (t, 6H), 0.83 (t, 3H).

### Comparative Example 9

### Preparation of Dimethylaminopropyl Cocoate (transesterification)

To a 100 mL flask fitted with a distillation head and condenser was added methyl cocoate (10 g, 0.0467 mol) and dimethylaminopropanol (5.77 g, 0.0561 mol, 1.2 eq). To the mixture was added stannous oxalate (0.103 g, 1 mol%). The flask was heated to 100 °C slowly over 1 hour. Over several hours the temperature was increased to 130 °C. The reaction was monitored by GC/MS. Methanol was collected in the receiver (ca. 1 mL). The reaction was allowed to cool to room temperature. The mixture was filtered to afford the product as a golden oil (10 g). ¹H NMR (300 MHz, CDCl₃) δ 7.02 (s, 1H), 3.28 (m, 2H), 2.32 (m, 2H), 2.18 (s, 6H), 2.10 (t, 2H), 1.59 (m, 2H), 1.21 (s, 16H), 0.84 (t, 3H).

### Comparative Example 10

### Preparation of Coconut Fatty Acid

To a 2 L flask was added coconut oil (100 g), methanol (435 mL) and water (307 mL). To this mixture was added 45% potassium hydroxide (88 g). The solution was heated at 45 °C overnight. The reaction was monitored by GC/MS. When the reaction was complete, the mixture was allowed to come to room temperature. To the flask was added methanol (275 mL) and heptane (200 mL). The mixture was stirred and transferred to a separatory funnel. The aqueous layer was returned to the 2 L flask. The organic layer was discarded. To the flask was added water (50 mL). The pH was brought to 1 with the addition of concentrated HCl (ca. 70 mL). The mixture was stirred well and transferred to a separatory funnel. The aqueous layer was removed. The organic layer was dried over MgSO₄ and concentrated *in vacuo* to afford the product as a yellow oil (80 g). ¹H NMR (300 MHz, CDCl₃) δ 11.68 (s, 1H), 2.36 (t, 2H), 1.65 (m, 2H), 1.28 (s, 16H), 0.90 (t, 3H).

### Comparative Example 11

### Preparation of Dimethylaminopropyl Cocoate (direct esterification)

To a 100 mL flask fitted with a distillation head and condenser was added coconut fatty acid (10 g, 0.05 mol,) and dimethylaminopropanol (6.18 g, 0.06 mol, 1.2 eq). The flask was heated to 40 °C (under nitrogen) to melt the fatty acid. To the molten mixture was added stannous oxalate (0.103 g, 1 mol%). The flask was heated to 100 °C slowly over 1 hour. Over several hours the temperature was increased to 150 °C. The reaction was monitored by GC/MS. Water was collected in the receiver (ca. 1 mL). The reaction mixture was allowed to cool to room temperature. The mixture was diluted with diethyl ether and washed with saturated sodium bicarbonate solution. The organic layer was dried and concentrated *in vacuo* to afford the product as a yellow oil (2.6 g). ¹H NMR (300 MHz, CDCl₃) δ 7.02 (s, 1H), 3.28 (m, 2H), 2.32 (m, 2H), 2.18 (s, 6H), 2.10 (t, 2H), 1.59 (m, 2H), 1.21 (s, 16H), 0.84 (t, 3H).

## Claims

1. A process for the preparation of a betaine, comprising:
a) producing an ester of formula 2: 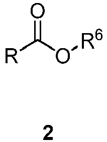
b) reacting a dialkylamino alcohol 3: 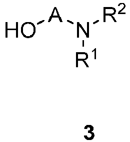 with 2 in the presence of an enzyme Candida antartica lipase B immobilized on acrylic resin to form an intermediate 4: 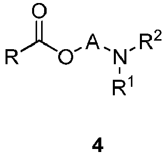
c) reacting intermediate 4 with sodium chloroacetate to produce a betaine;
wherein R is selected from the group consisting of branched and straight-chain, saturated, unsaturated and polyunsaturated C₁-C₂₂ hydrocarbyl, C₃-C₈ cycloalkyl, C₆-C₂₀ carbocyclic aryl, and C₄-C₂₀ heterocyclic wherein the heteroatoms are selected from the group consisting of sulfur, nitrogen, oxygen, and mixtures thereof,
R⁶ is a straight or branched chain C₁-C₆ alkyl,
R¹ and R² are the same or are independently selected from the group consisting of straight or branched-chain C₁-C₆ alkyl, C₂-C₆ alkenyl, C₄-C₆ dienyl, and C₃-C₈ cycloalkyl, and
A is selected from the group consisting of branched and straight-chain, saturated, unsaturated and polyunsaturated C₁-C₁₀ divalent hydrocarbyl, C₃-C₈ cycloalkylene, C₆-C₁₀ carbocyclic arylene, and C₄-C₁₀ divalent heterocyclic wherein the heteroatoms are selected from sulfur, nitrogen, and oxygen;
and wherein said hydrocarbyl group in R and/or said divalent hydrocarbyl group in A may be substituted with one to five groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen;
and wherein said alkyl, alkenyl and dienyl groups in R¹ and R² may be substituted with one to three groups selected from C₁-C₆-alkoxy, carboxyl, amino, C₂-C₁₆ aminocarbonyl, C₂-C₁₆ amido, cyano, C₂-C₇-alkoxycarbonyl, C₂-C₇-alkanoyloxy, hydroxy, aryl, heteroaryl, thiol, thioether, C₂-C₁₀ dialkylamino, C₃-C₁₅ trialkylammonium and halogen, wherein the branching and/or substitution of R¹ and R² may connect to form a ring.

2. The method according to claim 1, wherein the ester is produced by solvolysis of triglycerides in the presence of a C₁-C₄ alcohol and a base, acid or enzyme catalyst.

3. The method according to claim 2, wherein the C₁-C₄ alcohol is methanol, ethanol, 1-propanol, 2-propanol, 1-butanol, 2-butanol, or isobutanol.

4. The method according to claim 1 wherein the betaine is prepared in water, a C₁-C₄ alcohol, or a C₁-C₄ diol.

5. The method according to claim 4 wherein the C₁-C₄ alcohol is isopropanol.

6. The method according to claim 4 wherein the C₁-C₄ diol is 1,3-propanediol.

7. The method of any of the preceding claims further comprising including the betaine as a surfactant in a formulation to produce a formulated product.

8. The method of claim 7 wherein said betaine is present in an amount of from 0.001 weight % to 20 weight %.

9. The method of claim 7 wherein said betaine is present in an amount of from 0.01 weight % to 15 weight %.

10. The method of claim 7 wherein said betaine is present in an amount of from 0.1 weight % to 10 weight %.

11. The method of claims 7 to 10 wherein the formulated product is from skin care, hair care or other cosmetic products, household and industrial surface cleaners, disinfectants, metal working, rust inhibitors, lubricants, agrochemicals, and dye dispersions.

## Patentansprüche

1. Verfahren zur Herstellung eines Betains, umfassend:
a) Herstellung eines Esters der Formel 2: 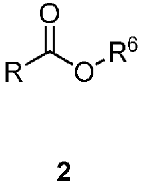
b) Umsetzung eines Dialkylamino-Alkohols 3: 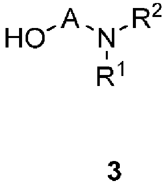 mit 2 in der Gegenwart eines Enzyms Candida antartica lipase B, immobilisiert auf einem Acrylkunststoff, um ein Zwischenprodukt 4 zu bilden: 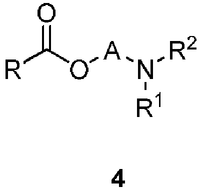
c) Umsetzung von Zwischenprodukt 4 mit Natriumchloracetat, um ein Betain zu erhalten;
wobei R ausgewählt ist aus der Gruppe, bestehend aus verzweigtem und geradkettigem, gesättigtem, ungesättigtem und polyungesättigtem C₁-C₂₂-Kohlenwasserstoff, C₃-C₈-Cycloalkyl, C₆-C₂₀-Carbocyclischaryl und C₄-C₂₀-heterocyclisch, wobei die Heteroatome ausgewählt sind aus der Gruppe, bestehend aus Schwefel, Stickstoff, Sauerstoff und Mischungen davon,
R⁶ ist ein geradkettiges oder verzweigtkettiges C₁-C₆-Alkyl,
R¹ und R² sind gleich oder unabhängig voneinander ausgewählt aus der Gruppe, bestehend aus geradkettigem oder verzweigtkettigem C₁-C₆-Alkyl, C₂-C₆-Alkenyl, C₄-C₆-Dienyl und C₃-C₈-Cycloalkyl, und
A ist ausgewählt aus der Gruppe, bestehend aus verzweigtkettigem und geradkettigem, gesättigtem, ungesättigtem und polyungesättigtem C₁-C₁₀-divalentem Kohlenwasserstoff, C₃-C₈-Cycloalkylen, C₆-C₁₀-carbocyclischem Arylen und C₄-C₁₀-divalent heterocyclisch, wobei die Heteroatome ausgewählt sind aus Schwefel, Stickstoff und Sauerstoff;
und wobei die Kohlenwasserstoffgruppe in R und/oder die divalente Kohlenwasserstoffgruppe in A substituiert sein kann mit ein bis fünf Gruppen, ausgewählt aus C₁-C₆-Alkoxy, Carboxyl, Amino, C₂-C₁₆-Aminocarbonyl, C₂-C₁₆-Amido, Cyano, C₂-C₇-Alkoxycarbonyl, C₂-C₇-Alkanoyloxy, Hydroxy, Aryl, Heteroaryl, Thiol, Thioether, C₂-C₁₀-Dialkylamino, C₃-C₁₅-Trialkylammonium und Halogen;
und wobei die Alkyl-, Alkenyl- und Dienylgruppen in R¹ und R² substituiert sein können mit ein bis drei Gruppen ausgewählt aus C₁-C₆-Alkoxy, Carboxyl, Amino, C₂-C₁₆-Aminocarbonyl, C₂-C₁₆-Amido, Cyano, C₂-C₇-Alkoxycarbonyl, C₂-C₇-Alkanoyloxy, Hydroxy, Aryl, Heteroaryl, Thiol, Thioether, C₂-C₁₀-Dialkylamino, C₃-C₁₅-Trialkylammonium und Halogen, wobei die Verzweigung und/oder Substitution von R¹ und R² unter Bildung eines Ringes verknüpfen kann.

2. Verfahren nach Anspruch 1, wobei der Ester durch Solvolyse der Triglyceride in der Gegenwart eines C₁-C₄-Alkohols und einer Base, Säure oder Enzymkatalysators hergestellt wird.

3. Verfahren nach Anspruch 2, worin der C₁-C₄-Alkohol Methanol, Ethanol, 1-Propanol, 2-Propanol, 1-Butanol, 2-Butanol oder Isobutanol ist.

4. Verfahren nach Anspruch 1, worin das Betain hergestellt wird in Wasser, einem C₁-C₄-Alkohol oder einem C₁-C₄-Diol.

5. Verfahren nach Anspruch 4, worin der C₁-C₄-Alkohol Isopropanol ist.

6. Verfahren nach Anspruch 4, worin das C₁-C₄-Diol 1,3-Propandiol ist.

7. Verfahren nach irgendeinem der vorhergehenden Ansprüche, weiterhin umfassend das Einschließen des Betains als ein Tensid in einer Formulierung, um ein formuliertes Produkt zu erzeugen.

8. Verfahren nach Anspruch 7, worin das Betain in einer Menge von 0,001 Gew.-% bis 20 Gew.-% vorliegt.

9. Verfahren nach Anspruch 7, worin das Betain in einer Menge von 0,01 Gew.-% bis 15 Gew.-% vorliegt.

10. Verfahren nach Anspruch 7, worin das Betain in einer Menge von 0,1 Gew.-% bis 10 Gew.-% vorliegt.

11. Verfahren nach Ansprüchen 7 bis 10, wobei das formulierte Produkt von Hautpflege-, Haarpflege- oder anderen kosmetischen Produkten, Haushalts- und Industrieoberflächenreinigern, Desinfektionsmitteln, Metallbearbeitung, Rostinhibitoren, Schmiermitteln, Agrochemikalien und Farbstoffdispersionen ist.

## Revendications

1. Procédé pour la préparation d'une bétaïne, comprenant :
a) la production d'un ester de formule 2 : 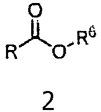
b) la réaction d'un dialkylaminoalcool 3 : 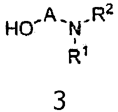 avec 2 en présence d'une enzyme lipase B de candida antartica immobilisée sur une résine acrylique pour former un intermédiaire 4 : 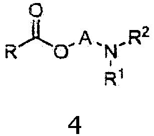
c) la réaction de l'intermédiaire 4 avec le chloroacétate de sodium pour produire une bétaïne ;
où R est choisi dans le groupe consistant en C₁-C₂₂ hydrocarbyle saturé, insaturé et polyinsaturé ramifié et linéaire, C₃-C₈ cycloalkyle, C₆-C₂₀ aryle carbocyclique et C₄-C₂₀ hétérocyclique où les hétéroatomes sont choisis dans le groupe consistant en le soufre, l'azote, l'oxygène, et leurs mélanges,
R⁶ est un C₁-C₆ alkyle linéaire ou ramifié,
R¹ et R² sont identiques ou sont choisis indépendamment dans le groupe consistant en C₁-C₆ alkyle linéaire ou ramifié, C₂-C₆ alcényle, C₄-C₆ diényle et C₃-C₈ cycloalkyle, et
A est choisi dans le groupe consistant en C₁-C₁₀ hydrocarbyle divalent saturé, insaturé et polyinsaturé ramifié et linéaire, C₃-C₈ cycloalkylène, C₆-C₁₀ arylène carbocyclique et C₄-C₁₀ hétérocyclique divalent où les hétéroatomes sont choisis parmi le soufre, l'azote et l'oxygène ;
et où ledit groupe hydrocarbyle dans R et/ou ledit groupe hydrocarbyle divalent dans A peuvent être substitués avec un à cinq groupes choisis parmi C₁-C₆-alcoxy, carboxyle, amino, C₂-C₁₆ aminocarbonyle, C₂-C₁₆ amido, cyano, C₂-C₇-alcoxycarbonyle, C₂-C₇-alcanoyloxy, hydroxy, aryle, hétéroaryle, thiol, thioéther, C₂-C₁₀ dialkylamino, C₃-C₁₅ trialkylammonium et halogène ;
et où lesdits groupes alkyle, alcényle et diényle dans R¹ et R² peuvent être substitués avec un à trois groupes choisis parmi C₁-C₆-alcoxy, carboxyle, amino, C₂-C₁₆ aminocarbonyle, C₂-C₁₆ amido, cyano, C₂-C₇-alcoxycarbonyle, C₂-C₇-alcanoyloxy, hydroxy, aryle, hétéroaryle, thiol, thioéther, C₂-C₁₀ dialkylamino, C₃-C₁₅ trialkylammonium et halogène, où la ramification et/ou substitution de R¹ et R² peuvent se lier pour former un cycle.

2. Procédé selon la revendication 1, où l'ester est produit par solvolyse de triglycérides en présence d'un C₁-C₄ alcool et d'un catalyseur base, acide ou enzyme.

3. Procédé selon la revendication 2, où le C₁-C₄ alcool est le méthanol, l'éthanol, le 1-propanol, le 2-propanol, le 1-butanol, le 2-butanol ou l'isobutanol.

4. Procédé selon la revendication 1 où la bétaïne est préparée dans l'eau, un C₁-C₄ alcool ou un C₁-C₄ diol.

5. Procédé selon la revendication 4 où le C₁-C₄ alcool est l'isopropanol.

6. Procédé selon la revendication 4 où le C₁-C₄ diol est le 1,3-propanediol.

7. Procédé selon l'une quelconque des revendications précédentes comprenant en outre l'inclusion de la bétaïne comme tensioactif dans une formulation pour produire un produit formulé.

8. Procédé selon la revendication 7 où ladite bétaïne est présente en une quantité de 0,001 % en poids à 20 % en poids.

9. Procédé selon la revendication 7 où ladite bétaïne est présente en une quantité de 0,01 % en poids à 15 % en poids.

10. Procédé selon la revendication 7 où ladite bétaïne est présente en une quantité de 0,1 % en poids à 10 % en poids.

11. Procédé selon les revendications 7 à 10 où le produit formulé est parmi les produits de soins pour la peau, de soins pour les cheveux ou d'autres produits cosmétiques, les agents de nettoyage de surface ménagers et industriels, les désinfectants, les agents de travail des métaux, les inhibiteurs de rouille, les lubrifiants, les produits agrochimiques et les dispersions de colorants.
